# EUROPÄISCHE PATENTANMELDUNG

(11) **EP 1 770 392 A1**
(43) Veröffentlichungstag der Anmeldung: **04.04.2007**
(21) Anmeldenummer: 06019912.2
(22) Anmeldetag: 22.09.2006
(51) Int. Cl.: G01N 27/414, G01N 33/00

(54) **Gassenitiver Feldeffekttransistor zur Detektion von Chlor**

(30) Priorität: 30.09.2005 DE 102005046944
(71) Anmelder: Micronas GmbH, 79108 Freiburg i. Br. (DE)
(72) Erfinder: Knittel, Thorsten, 93080 Pentling/Grossberg (DE); Freitag, Gunter, 81735 München (DE); Eisele, Ignaz, 82057 Icking (DE)
(74) Vertreter: Göhring, Robert

(57) **Zusammenfassung**

Gassensitiver Feldeffekttransistor, der nach dem Prinzip der Austrittsarbeitsmessung generierte Signale ausliest, zur Detektion von Chlor (C1) mit einer gassensitiven Schicht,
wobei die gassensitive Schicht im Wesentlichen aus Gold besteht.

## Beschreibung

### Gassensitiver Feldeffekttransistor zur Detektion von Chlor

Die Erfindung betrifft einen Gassensor zur Detektion von Chlor (Cl).

Die Detektion von Chlor, insbesondere in der Umgebungsluft, ist aufgrund der toxischen bzw. ätzenden Eigenschaften dieses Gases sinnvoll. Um diese Messungen jedoch akzeptabler zu gestalten, muss die ausreichende Selektivität mit einfach aufgebauten Sensoren kombiniert werden.

Bekannte Gassensoren für Chlor basieren bisher in der Regel auf elektrochemischen Zellen, die bei relativ hohem Preis lediglich eine kurze Lebensdauer aufweisen und darüber hinaus hohe Konzentrationen nur bedingt messen können. Chlor an sich ist ein hochtoxisches Gas, welches darüber hinaus stechend riecht. Werte für die maximale Arbeitsplatzkonzentration (MAK) liegen bei 10 vpm mit momentanem Spitzenwert von 20 vpm.

Wegen der niedrigen Geruchsschwelle, bzw. des stechenden Geruches wird Chlor bereits in sehr kleinen Konzentrationen wahrgenommen, so dass es auch als Leitgas für die Luftgüte herangezogen werden kann, beispielsweise bei der Regelung für Kraftfahrzeugklimaanlagen.

Für viele Anwendungen werden preisgünstige Sensoren verlangt, die zwar nur sehr niedrige Schwellwerte bei der Ermittlung der Cl-Konzentration berücksichtigen sollen, dies jedoch mit hoher Selektivität und Reproduzierbarkeit. Der Leistungsbedarf soll derart gering sein, dass ein mehrmonatiger Batteriebetrieb bzw. direkter Anschluss beispielsweise ohne Hilfsenergie an eine Datenbusleitung möglich ist.

Aufgrund der hohen Bedeutung für die Sicherheit und aufgrund des breiten Einsatzbereiches der Chlormessungen sind bereits heute eine große Zahl unterschiedlicher Messsysteme im Einsatz. Verwendet werden sensitive elektrochemische Zellen. Deren Preis ist jedoch für viele Anwendungen viel zu hoch. Darüber hinaus benötigen darauf aufgebaute Sensorsysteme einen hohen Wartungsaufwand. Die Lebensdauer der einzelnen Sensoren ist relativ kurz.

Im unteren Preissegment sind die Metalloxidsensoren vertreten. Deren Reaktion auf ein Zielgas wird über Leitfähigkeits-änderungen entsprechend ausgelesen. Diese Sensoren werden jedoch bei höheren Temperaturen betrieben wie beispielsweise oberhalb von 200° C. Somit benötigen derartige Sensoren eine hohe Leistung, um die Betriebstemperatur zu erreichen. Daraus folgt direkt, dass derartige Sensoren für viele Anwendungsfälle, wie batteriebetriebene Systeme oder für den direkten Anschluss an einen Datenbus nicht geeignet sind.

Aufgrund gesetzlicher Auflagen wird der Einsatz von Chlorsensoren jedoch ständig erhöht. Dabei bestehen im Wesentlichen Nachteile durch hohe Sensorkosten durch die Versorgung der Sensoren mit der erforderlichen Betriebsenergie und ähnliches. Außerhalb von gesetzlich vorgeschriebenen Anwendungen werden aus den genannten Gründen Chlorsensoren in der Praxis lediglich dann eingesetzt, wenn dies beispielsweise für die Regelung von Geräten oder Anlagen unabdingbar ist und die Betriebsenergie ohne weiteren Aufwand zur Verfügung steht.

Gassensoren, die die Änderung der elektronischen Austrittsarbeit bzw. deren Veränderung an Materialien bei der Wechselwirkung mit zu detektierenden Gasen als sensitives Messprinzip nutzen, sind geeignet, bei niedrigen Temperaturen und damit niedrigem Energieaufwand betrieben zu werden. Es wird die Möglichkeit ausgenutzt, die Änderung der Austrittsarbeit von gassensitiven Materialien in einem Feldeffekttransistor (GasFET) einzukoppeln und dadurch die Änderung der Austrittsarbeit als Änderung des Stromes zwischen Source und Drain des Transistors zu messen.

Im Wesentlichen werden zwei Transistortypen als so genannte GasFET eingesetzt. Dies ist zum einen der so genannte Suspended Gate Fieldeffect Transistor (SGFET) und zum anderen der Capacitively Coupled Fieldeffect Transistor (CCFET). Bei beiden Typen liegt eine abgehobene Gate-Elektrode unter Ausbildung eines Luftspaltes der Chipoberfläche gegenüber. Beim SGFET ist der Kanalbereich des Transistors an der der gassensitiven Schicht gegenüber liegenden Seite des Luftspalts angeordnet und mittels einer den Kanalbereich bedeckenden geeigneten Isolationsschicht von diesem getrennt.

Beim CCFET befindet sich der Luftspalt zwischen der gassensitiven Schicht und einer gegenüberliegenden Elektrode, die kapazitiv an die gassensitive Schicht koppelt.

Diese Elektrode ist leitend mit dem Gate des Signal gebenden Feldeffekttransistors verbunden, wobei letzterer räumlich von dem Luftspalt getrennt sein kann. Die Elektrode kann zum Luftspalt hin mit einer geeigneten Isolationsschicht bedeckt sein.

Beide sind gekennzeichnet durch den hybriden Aufbau, der ein relativ einfaches und zuverlässiges Aufbauprinzip begründet. So kann hier das gasempfindliche mit der gassensitiven Schicht belegte Gate (Elektrode) einerseits und der eigentliche Transistor andererseits getrennt hergestellt werden, wobei die Vollendung des Aufbaus beispielsweise durch Flip-Chip-Technologie eine Verbindung beider Elemente unter gleichzeitig genauer gegenseitiger Positionierung ermöglicht. Ein Vorteil, der durch diese hybride Aufbautechnik direkt erzielbar ist, liegt in der Einsatzmöglichkeit vieler Materialien als gassensitive Schicht, wobei diese Materialien in der Regel aufgrund ihrer artfremden Zusammensetzung nicht mit den beispielsweise Siliziumkomponenten eines Feldeffekttransistors kombinierbar wären. Dies trifft insbesondere auf Metalloxide zu, die in Dick- oder Dünnschichttechnologie aufgebracht werden können.

Es sind bisher keine Materialien bekannt, mittels derer ein gassensitiver Feldeffekttransistor Chlor detektieren kann.

Aufgabe der Erfindung ist die Bereitstellung eines Chlorsensors, der durch einen FET auslesbar ist.

Die Lösung dieser Aufgabe geschieht durch die Kombination der Merkmale entsprechend Anspruch 1. Vorteilhafte Ausgestaltungen sind den Unteransprüchen zu entnehmen.

Die Erfindung beruht auch auf der Verwendung von Feldeffekttransistoren zur Auslesung der Austrittsarbeit an gassensitiven Schichten. Die dabei entstehenden gassensitiven Feldeffekttransistoren weisen Betriebstemperaturen auf, die zwischen Raumtemperatur und 200° C liegen können. Gewisse Temperaturschwankungen oder Temperaturerhöhungen sind notwendig, um reversible Änderungen ablaufen zu lassen. Die resultierende geringe Betriebstemperatur ermöglicht durch die Kombination mit auf Chlor ansprechende gassensitiven Schichten einen Gassensor, der die geforderten Bedingungen erfüllt. Nachdem für gassensitive Feldeffekttransistoren bisher keine gassensitiven Schichten zur Detektion von Chlor bekannt sind ist erkannt worden, dass Gold als gassensitives Material für die Detektion Chlor in Frage kommt. Nachdem Gold bei Kontakt mit Chlor Goldchlorid bildet und Gold und Goldchlorid verschiedene Austrittsarbeiten aufweisen, kann diese Reaktion und somit die Anwesenheit von kleinsten Chlorkonzentrationen mit einem gassensitiven FET nachgewiesen werden. Somit kann die Austrittsarbeitsdifferenz mit Hilfe eines Gassensors auf Feldeffektbasis ausgelesen und als Gassignal interpretiert werden.

Ein ungeheizter Sensor zeigt eine hohe Sensitivität und große Signalpegel. Das Signal ist jedoch nicht reversibel.

Dieser bei Zimmertemperatur betriebene Sensor kann bis in den hohen ppb-Bereich (parts per billion) auflösen. Diese Sensorvariante kann als dosimetrischer Sensor verwendet werden, das Anzeigesignal ist dann die Chlordosis, also das aufsummierte Produkt aus vorhandener Chlorkonzentration und der Zeit, in der diese Chlorkonzentration anwesend ist. Nach einer gewissen Dosis, muß der Sensor dann ersetzt, oder wie unten regeneriert werden.

Der Sensor kann jedoch nach der Messung durch eine kurze Heizperiode bei ca. 200°C oder mehr reaktiviert werden, d.h. es erfolgt eine Rückstellung des Signals.

Alternativ kann der Sensor konstant beheizt betrieben werden. Dann erfolgt eine kontinuierliche Rückstellung des Signals, was dazu führt, das das Sensorsignal der aktuell vorhandenen Chlorkonzentration folgt. Mit zunehmender Betriebstemperatur verlagert sich die Sensitivität des Sensors zu kleineren Konzentrationen von Chlor.

Der Vorteil des beschriebenen Sensorprinzips liegt darin, dass ein miniaturisierter und kostengünstiger Sensor angegeben wird, der zugleich Langzeitstabilität aufweist, d. h. nicht eine immanente Begrenzung der Lebensdauer auf maximal ca. 2 Jahre aufweist. Durch die Möglichkeit, diesen Sensor auch ungeheizt als Dosimeter zu betreiben, kann er auch ohne benötigte Heizenergie in mobilen Anwendungen betrieben werden).

Im Folgenden werden anhand von schematischen, die Erfindung nicht einschränkenden Figuren Ausführungsbeispiele beschrieben.
- Figur 1: zeigt den schematischen Aufbau eines GasFETs in der speziellen Ausführung eines SGFET,
- Figur 2: zeigt Chlorgas-Belastung und Signale eines gassensitiven FETs bei Raumtemperatur,

- Figur 3: zeigt ein analoges Diagramm zu Fig 2 mit Signalen bei 80°C,
- Figur 4: zeigt ein analoges Diagramm zu Fig 2 mit Signalen bei 180°C.

Durch die Erfindung ergeben sich insbesondere folgende Vorteile:
- Der Betrieb mit geringem Energieverbrauch wird ermöglicht,
- die geringe geometrische Größe, die eine Realisierung von Sensoranordnungen erleichtert, kann dargestellt werden,
- eine mögliche monolithische Integration der Elektronik in den Sensorchip kann realisiert werden,
- die Verwendung ausgereifter kostengünstiger Verfahren der Halbleiterfertigung ist zur Herstellung eines entsprechenden GasFETs möglich.

Die Anwendungs- und Einsatzgebiete von Chlorsensoren sind zahlreich, wobei viele aktuelle Probleme durch den vorteilhaften Einsatz der erfindungsgemäßen GasFETs in den Bereichen wie Kraftfahrzeugklimaanlage, Raumluftgüteüberwachung, Batteriebetrieb von Geräten, insbesondere mobilen Geräten z.B. als persönliches tragbares Dosimeter für Arbeitsplatzsicherheit, und vernetzte Systeme, die Gassensoren über Datenbusleitungen organisieren, gelöst werden können.

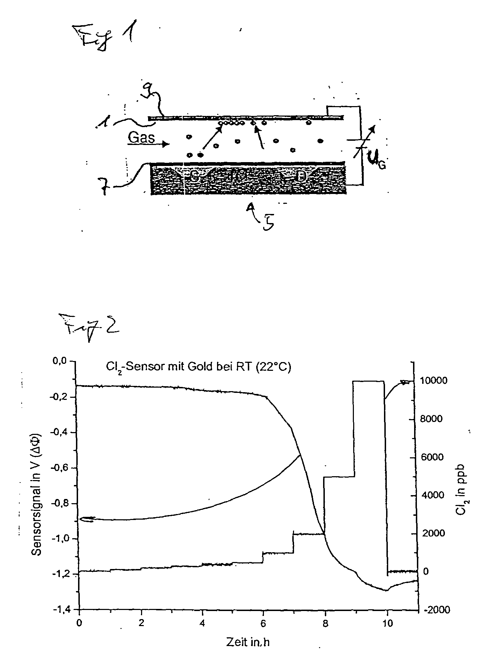

Figur 1 zeigt den Grundaufbau eines Gassensors mit abgehobenem Gate (SGFET), wobei die sensitive Schicht 1 auf der Gate-Elektrode 9 aufgebracht ist. Die Gate-Isolierung 7 gehört zum Basistransistoraufbau bestehend aus Transistorkanal 5 sowie benachbarter so genannter Source und Drain. Die Spannung U_{G} ist die so genannte Gate-Spannung, die im Zusammenhang mit einem Sensorsignal ausgebildet wird.

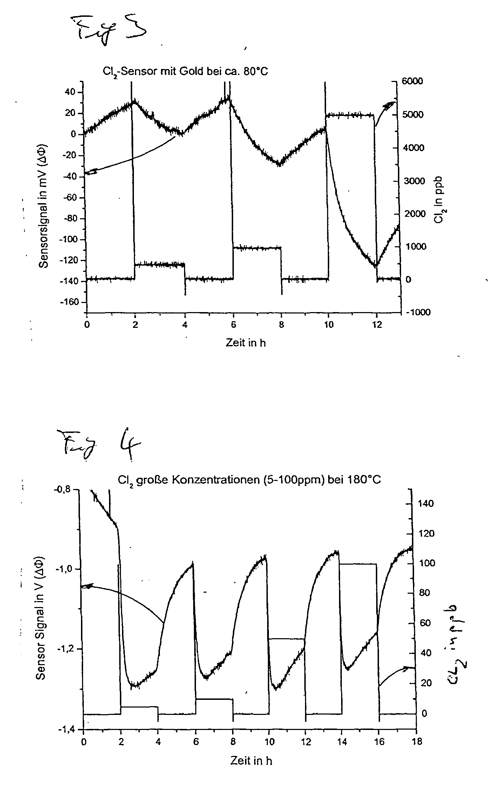

Figur 2 zeigt den Nachweis der Funktionsfähigkeit eines gassensitiven Feldeffekttransistors mit einer gassensitiven Schicht aus Gold. Diese Diagramme geben bei Raumtemperatur aufgenommene Messsignale wieder, beispielsweise bei 22°C. Die Funktionsweise basiert auf der Reaktion des Goldes bei der Anwesenheit von Chlor zu Goldchlorid. Diese Reaktion kann bei ca. 200°C wieder umgekehrt werden. Wird ein Sensor bei Raumtemperatur betrieben, so muss die entsprechende Betriebsweise eine zwischen geschaltete Regenerierungsphase mit einer kurzen Aufheizung auf ca. 200°C vorsehen Wird der Sensor beispielsweise bei ca. 150 - 180°C betrieben, so liegt ein reversibler, kontinuierlich funktionierender Sensor mit einer annehmbaren Zeitkonstanten vor. Reversibilität kann bereits bei ca. 80 °C erreicht werden.

Das Verhältnis zwischen Gold und Goldchlorid stellt sich in Abhängigkeit von der Gaskonzentration ein und kann mit dem GasFET bestimmt werden.

Figur 3 zeigt ein Sensorsignal, welches bei einer Betriebstemperatur von 80 °C aufgenommen ist. Die Signale sind bereits reversibel, die Zeitkonstante jedoch noch sehr hoch.

Figur 4 zeigt ein Diagramm mit Sensorsignalen, die bei 180°C Betriebstemperatur aufgenommen sind. Der Sensor ist voll reversibel. Konzentrationen von mehr als 5 ppm können nicht mehr aufgelöst werden. Für kleinste Chlorkonzentrationen ist der Sensor jedoch bestens geeignet.

Für die Präparation der gassensitiven Gold-Schichten können sowohl Kathodenzerstäubung, Aufdampfverfahren, Siebdruckverfahren, als auch CVD-Verfahren eingesetzt werden. Typische Schichtdicken liegen im Bereich zwischen 10 nm und 10 µm. Besonders vorteilhaft ist die Anwendung einer porösen offenporigen Schicht. Die Präparation von Gold oder Goldhaltigen Materialien in einem Gassensor zur Chlordetektion erweitert die Palette der Materialien für gassensitive Schichten, die in gassensitiven Feldeffekttransistoren verwendet werden. Neben der kostengünstigen Herstellung der Sensoren sind deren selektive und reproduzierbare Signale als Vorteile zu nennen.

Die Beheizung der Schicht ist teilweise erforderlich, damit nach der Gasbeaufschlagung eine Rückkehr zu einem Ursprungswert möglich ist. Der Betrieb des Sensors bei Raumtemperatur zeigt ein integrierendes Verhalten, wobei ab 80° C die Reaktion im Feldeffekttransistor reversibel ist; die Zeitkonstante ist jedoch noch relativ groß. Bei höheren Temperaturen reduziert sich allgemein der Signalpegel.

Die Funktionsweise von Gassensoren auf FET-Basis ist allgemein bekannt.

Somit stehen durch die Erfindung kostengünstige Chlorsensoren mit geringem Energiebedarf zur Verfügung, die durch ihre vorteilhaften Eigenschaften viele Einsatzgebiete bzw. Verwendungen abdecken.

## Patentansprüche

1. Gassensitiver Feldeffekttransistor, der nach dem Prinzip der Austrittsarbeitsmessung generierte Signale ausliest, zur Detektion von Chlor (Cl) mit einer gassensitiven Schicht,
**dadurch gekennzeichnet, dass**
die gassensitive Schicht im Wesentlichen aus Gold besteht.

2. Gassensitiver Feldeffekttransistor nach Anspruch 1, bei dem der Feldeffekttransistor ein SGFET (suspended gate FET) ist.

3. Gassensitiver Feldeffekttransistor nach Anspruch 1, bei dem der Feldeffekttransistor ein CCFET (capacitively coupled FET) ist.

4. Gassensitiver Feldeffekttransistor nach einem der Ansprüche 1, 2 oder 3, bei dem die Schichtdicken der gassensitiven Schicht zwischen 10nm und 10 µm stark sind.

5. Gassensitiver Feldeffekttransistor nach einem der Ansprüche 1 bis 4, bei dem die Betriebstemperatur zumindest der gassensitiven Schicht durch eine elektrische Heizung einstellbar ist.

6. Gassensitiver Feldeffekttransistor nach einem der Ansprüche 1 bis 5, der zum Chlor-Nachweis in einem mobilen batteriebetriebenen Gerät eingebaut ist.

7. Gassensitiver Feldeffekttransistor nach einem der vorhergehenden Ansprüche, der auf einem Halbleiterchip zusammen mit einer integrierten Schaltung angeordnet ist, wobei die integrierte Schaltung zur Vorverarbeitung, Analyse und Weitergabe der Signalen des gassensitiven FETs ausgelegt ist.

8. Verfahren zum Betrieb eines gassensitiven Feldeffekttransistors zur Detektion von Chlor nach einem der Ansprüche 1 bis 7, bei dem die gassensitive Schicht bei Raumtemperatur betrieben wird und zeitweise zur Regenerierung auf ca. 200°C aufgeheizt wird.

9. Verfahren zum Betrieb eines gassensitiven Feldeffekttransistors zur Detektion von Chlor nach einem der Ansprüche 1 bis 7, bei dem die gassensitive Schicht im Bereich von ca. 120 - 200 °C betrieben wird, wodurch ein reversibler Gassensor dargestellt ist.

10. Verwendung eines gassensitiven Feldeffekttransistors zur Detektion von Chlor, der entsprechend einem der Ansprüche 1 bis 9 aufgebaut ist bzw. betrieben wird, zur Überwachung der Einhaltung maximal erlaubter Grenzwerte hinsichtlich der Luftgüte in Aufenthaltsräumen.

11. Verwendung eines gassensitiven Feldeffekttransistors zur Detektion von Chlor, der entsprechend der Ansprüche 1 bis9 aufgebaut ist, bzw. betrieben wird, zum Einsatz in vernetzten Systemen.

12. Verwendung eines gassensitiven Feldeffekttransistors zur Detektion von Chlor, der entsprechend der Ansprüche 1 bis 9 aufgebaut ist bzw. betrieben wird, zur Detektion von austretendem Chlorgas in Anlagen, die Chlorgas lagern, verarbeiten oder enthalten oder bei deren Betrieb Chlorgas entstehen kann.
